# EUROPEAN PATENT APPLICATION

(11) **EP 2 096 123 A1**
(43) Date of publication of application: **02.09.2009**
(21) Application number: 07800858.8
(22) Date of filing: 04.09.2007
(51) Int. Cl.: C07K 19/00

(54) **A FUSION PROTEIN CARRYING NEUROTROPHIN ACROSS THE BLOOD-BRAIN BARRIER, ENCODING GENE AND UESE THEREOF**

(30) Priority: 25.09.2006 CN 200610122393
(71) Applicant: Southern Medical University Zhujing Hospital, Guangzhou 510282 (CN)
(72) Inventor: JI, Aimin, Guangzhou, 510282 (CN); CHE, Ou, Guangzhou, 510282 (CN); MA, Leilei, Guangzhou, 510282 (CN); SU, Dan, Guangzhou, 510282 (CN); MA, Hanzhang, Guangzhou, 510282 (CN); LI, Xiaodong, Guangzhou, 510282 (CN); SUN, Liang, Guangzhou, 510282 (CN); WANG, Bingjun, Guangzhou, 510282 (CN); ZHANG, Zhongyi, Guangzhou, 510282 (CN)
(74) Representative: Viering, Hans-Martin
(86) International application number: PCT/CN2007/002641
(87) International publication number: WO 2008/043241

(57) **Abstract**

Provided are fusion proteins which comprise a first region having at least 75% homology with the amino acid sequence of neurotrophin, and a second region, located at the C-terminal of the first region, having at least 75% homology with the amino acid sequence of protein transduction domain (PTD). Also provided is encoding gene and usage thereof. The fusion protein can translocate through cell membrane or even the blood-brain barrier (BBB). The fusion protein can be used to treat various central nervous system degenerative diseases and peripheral neuropathy.

## Description

### TECHNICAL FIELD

This invention relates to neurotrophins fusion proteins, and particularly to the fusion proteins containing neurotrophins and protein transduction domains (PTD), and their coding genes, expression vectors, host cells, and uses.

### BACKGROUND OF THE INVENTION

Neurotrophins (NTs) are low molecular weight protein factors capable of promoting the survival, growth and differentiation of nerve cells. During the development of nervous system, nerve cells which get enough neurtrophic factors will survive, and others will undergo natural death. Nervous system development is always companied by nerve cells' death. Some unnatural death (for example, some neurodegenerative diseases), may be reduced by obtaining the external NTs. NTs have become one of the most interesting research fields in neuroscience.

NTs of mammalian include nerve growth factor (NGF), brain-derived neurotrophic factor (BDNF), neurotrophin 3(NT-3) and neurotrophin 4/5(NT-4/5). Each member is capable of forming dimmer structure and binding specifically to particular receptors. The binding makes the receptors dimerize and autophosphorylate, thus activates several downstream effector molecules and signaling pathways, thereby provides trophic effect for the survival and growth of the nerve cells (See, for example, Zweifei LS, et al. Function and mechanisms of retrograde neurotrophin signaling. Nature Review Neuroscience. 2005;6:615-625).

The NTs family members have some common characteristics in structure, including similar molecular weight (13.2-15.9 kDa); near or more than 50% homology in the primary structure of the amino acid sequences; the isoelectric points between about 9 and about 10; and three intramolecular disulfide bonds formed by six cysteine residues at the same sites. Subsequent to the hydrophobic region signal peptides of the N-terminal of NTs precursor protein are continuous basic amino acid residues. Sequence analysis shows digestion of NTs precursor proteins (with molecular weight of about 31-35 kDa) into the maturation proteins by the precursor proteins transferase.

As a polypeptide, NTs can not cross the blood-brain barrier (BBB), which is composed of vascular endothelial cells, when administered by peripheral vascular route, or can not reach an effective concentration. Therefore, NTs can only protect the neurons when injected directly into the cerebral ischemic area or cerebrospinal fluid. However, in clinic, these administration methods not only increase the technical demands to medical staff, but also bring new wounds that are unacceptable by patients. So, new administration methods are the precondition for NTs' clinical use (Miller G. Breaking down barriers. Science.2002;297:1116-1118).

The earliest method for medicines to cross BBB into brain used hyperosmotic glucose solution to dehydrate endothelial cells instantaneously, which caused the cells to contract, so as to break the endothelial cells barrier, and bring the medicines across the BBB to brain parenchyma. However, other components in blood, such as toxic components, will cross the BBB together with the targeted medicines. This makes the method still not be suitable for further clinical trial (Miller G. Breaking down barriers. Science. 2002; 297:1116-1118). Some researchers take the specific transporter protein receptors on BBB as the targets, such as transferrin receptor or insulin receptor, and have prepared MAbs-NTs conjugates or fusion protein. By administration through peripheral vascular route, these MAbs in the conjugate will be recognized by surface receptors on BBB and be endocytosed. After intracellular transport, the MAbs could escape from surface receptors on BBB next to brain parenchyma and get into the brain (with same mechanism as transferrin). The NTs in the conjugates also cross the BBB at the same time. But the conjugates in this method are not stable, and the fusion proteins produced by genetic engineering are with low yield and high cost. So this method is also not suitable for mass production (Zhang Y, et al. Neuroprotection in transient focal brain ischemia after delayed intravenous administration of brain-derived neurotrophic factor conjugated to a blood-brain barrier drug targeting system. Stroke. 2001;32:1378-1384).

### SUMMARY OF THE INVENTION

Due to the problems of prior art, the present invention provides a fusion protein for carrying neurotrophins across BBB and its nucleotide sequence, and preparation and uses thereof. The present invention addresses the problems associated with the unstable conjugates , low yield fusion proteins and entrance of toxic component into brain parenchyma due to use of hyperosmotic solution.

In one aspect, the invention provides a fusion protein for carrying neurotrophins across blood-brain barrier, which contains a first region having at least about 75% homology with the amino acid sequences of mature neurotrophin, and a second region having at least about 70% homology with the amino acid sequences of protein transduction domain (PTD), and wherein the second region is located at the carboxyl terminal of the first region.

In a preferred embodiment, the first region has at least about 85% homology with the amino acid sequences of mature neurotrophin, and the second region has at least about 85% homology with the amino acid sequences of protein transduction domain.

In a particularly preferred embodiment, the first region is a human nerve growth factor, brain-derived neurotrophic factor, neurotrophin 3, or neurotrophin 4/5.

In another preferred embodiment, the second region is from a homologous heterotypic protein Antp originated from fruit fly, from a pVEC of type I membrane protein Cadherin from mice vascular endothelial cells, or from transactivator of transcription (TAT) of human immunodeficiency virus 1 (HIV-1).

In a preferred embodiment, the second region is located immediately adjacent to the first region, or connects with the first region through intervening amino acids (or referred as a linker of oligopeptide).

In a more preferred embodiment, the first region is human brain-derived neurotrophic factor, and the second region is homologous heterotypic protein Antp originated from fruit fly, and thus the fusion protein has an amino acid sequence presented in SEQ ID NO: 1.

In another aspect, the invention provides a nucleotide sequence encoding the fusion protein. Said nucleotide sequence is as presented in SEQ ID NO: 2 or the complementary sequence of sequence of SEQ ID NO: 2.

In yet another aspect, the invention provides an expression vector comprising the nucleotide sequence having a sequence shown in SEQ ID NO: 2 or the complementary sequence thereof.

In still another aspect, the invention provides a host cell comprising said expression vector.

In still yet another aspect, the invention provides the use of fusion protein in the preparation of medicines for treating neurodegenerative diseases.

In one embodiment of the present invention provides fusion proteins in which one or several amino acid is substituted, deleted or inserted without any change in the characteristics of the fusion protein.

Also provided is a nucleotide sequence encoding the fusion protein, which includes sequences encoding PTD and NTs having biological activities, and the alleles, homolog, mutant of the sequences. The "protein transduction domains(PTD)" used herein refers to positive-charge-rich domains that can bring compounds, proteins or nucleic acids which are physically or chemically attached to the domain through BBB or cell membrane into cytoplasm, or even nucleus, allowing to exert their biological functions. The conjugates across the cell membrane mediated by PTD can be carried out without the need for energy or receptors-mediated way (Wadia JS, et al. Transducible TAT-HA fusogenic peptides enhances escape TAT-fusion proteins after lipid raft macropinocytosis. Nature Medicine. 2004;10(3):310-315). Some PTDs have been discovered, such as homologous heterotypic protein Antp originated from fruit fly (Eiriksdottir E, et al. Cellular uptake of cell-penetrating peptides. Drug Design. Online 1. 2004;161-173), pVEC of type I membrane protein Cadherin from mice vascular endothelial cells (Elmquist A, et al. VE-cadherin derived cell-penetrating peptide, pVEC, with carrier functions. Exp. Cell Res. 2001;269:237-244), and transactivator of transcription Tat originated from HIV-1.

Comparing with the prior art, one advantage of the present invention is to free the N-terminal of mature neurotrophins by the way of linking the second region with the C-terminal of the first region, which may ensure the right stereo conformation to keep the original bioactivity of neurotrophins.

### BRIEF DESCRIPTION OF THE DRAWINGS

Fig 1 is a graph showing human NTs-PTD/Antp nucleic acids;
Fig 2 is a graph showing the restriction endonuclease mapping of the expression vector for BDNF-PTD/Antp fusion protein;
Fig 3 is a graph showing the analysis of BDNF-PTD/Antp fusion protein by SDS-PAGE;
Fig 4 is a graph showing the analysis of BDNF-PTD/Antp fusion protein by Western blot;
Fig 5 is a graph showing the Western blot analysis of BDNF-Antp in brain after systemic administration;
Fig 6 is a graph showing the difference of biological activity between BDNF-PTD/Antp and BDNF in different concentrations;
Fig 7 is a graph showing the distribution of recombinant BDNF protein and recombinant BDNF-PTD/Antp fusion protein in brain after crossing the BBB;
Fig 8 is a graph showing the comparison of functional difference among BDNF-PTD/Antp fusion proteins in different structures;
Fig 9 is a graph showing the TTC staining of gerbil brain in cerebral ischemia model after intraperitoneal injection of BDNF-Antp;
Fig 10 is a graph showing the gray analysis of TTC staining in Fig.9;
Fig 11 is a graph showing the immunohistochemical staining for tyrosine hydroxylase in the substantia nigra of mice Parkinson's disease model induced by MPTP after intraperitoneal administration of BDNF-PTD/Antp fusion protein.

### DETAILED DESCRIPTION OF THE INVENTION

The highly basic domain (amino acid 41^{th}-59^{th}) within the antennapedia (Antp) is a positive-charge-rich domain, which is relevant to the transmembrane activity ( also referred to as protein transduction domain(PTD)(Snyder EL and Dowdy SF. Cell penetrating peptides in drug delivery. Pharm Res.2004;21(3):389-393)). For the purpose of the present invention, hereinafter, PTD derived from Antp is referred to as PTD/Antp. PTD/Antp can be fused to various full-length proteins or polypeptides ranging from 10 to 120 kDa, including ovalbumin, horseradish peroxidase(HRP), Cdk inhibitor p27^{Kipl} and the like (see, Ryu J, et al. Enhanced uptake of a heterologous protein with an HIV-1 tat protein transduction domains (PTD) at both termini. Mol Cells. 2003;16(3):385-391). Furthermore, PTD/Antp can deliver proteins to tissues cell in vivo, while assuring the biological activity of the proteins (Asoh S, et al. Protection against ischemic brain injury by protein therapeutics. Proc. Natl. Acad. Sci. USA. 2002; 99:17107-17112).

The fusion protein can deliver interest proteins across plasma membrane regardless of PTD/Antp been fused to the N-terminal or C-terminal of the fusion proteins. Appropriate fusion modes may be selected according to practical requirements. Proteins which are unable to cross the blood-brain barrier (BBB) under normal condition may cross the BBB by the way of PTD/Antp-mediation and function in the brain.

The present invention is based on an unexpected discovery. The fusion proteins with PTDs at the C-terminal of neurotrophins have better bioactivity than that with PTDs at the N-terminal. The possible reason may be that the full-length neurotrophin containing protein leader region located at the N-terminal of the neurotrophin can preferentially bind to the specific receptor p75 after forming a dimer by intermolecular disulfide bond, which activates the downstream signal transduction pathway and induces the neuronal death and apoptosis (Lu B, et al. The yin and yang of neurotrophin action. Nature Reviews Neuroscience. 2005;6:603-614; Teng HK, et al. ProBDNF induces neuronal apoptosis via activation of a NTR receptor complex of p75 and sortilin. J of Neuroscience. 2005;25(22):5455-5463). It is necessary to cut the precursor protein so as to form mature protein when neurotrophins (NTs) exhibit neurotrophic function. Mature protein region with the naked N-terminal can form intermolecular disulfide bonds. The newly formed dimer binds to a specific family member of the tyrosine protein kinase receptor, provoking the signal transduction of downstream pathway and thus exerting the neurotrophic function (Nature Reviews Neuroscience. 2005;6:603-614). PTD binding to the N-terminal of full-length neurotrophins will induces the death or apoptosis of neuron, and the PTD will be cut together with the leader protein when the full-length neurotrophins are cut. The absence of PTD will result in disability of crossing BBB of the mature protein. If PTD binding to the N-terminal of the mature protein, the N-terminal of the mature protein will not be naked, and thus no dimer will be formed. Therefore, the structure of naked N-terminal plays an important role in maintaining the correct stereo conformation for mature neurotrophin to bind with trk receptor.

The sequence of PTD is preferably immediately adjacent to NTs, or connects to NTs through intervening amino acid residues. The intervening amino acid residues may have 1-20 or 1-10 amino acids in length or even longer. Particularly preferably, one or more of the intervening amino acid residues are flexible amino acid residues. The NTs mature protein begins with Methionine. PTD connects to NTs in such a way that the PTD can fulfill the function of crossing the cell membrane or BBB, and the spatial stereo conformation of NTs and the formation of active dimer are not influenced by insertion of PTD or the intervening amino acids (in case of PTD been connected to NTs through intervening amino acids).

The nucleotide sequence encoding NTs and PTD can be native or synthetic, including allele gene, species homologue, mutant and variant. One or more nucleotides may be inserted, replaced or deleted without changing the function of the protein encoded. The nucleotide sequence encoding NTs and PTD can be obtained by conventional methods in the art, such as PCR (Saiki, et al. Science, 239:487-491, 1988), RT-PCR, artificial synthesis and cDNA library construction. The template for PCR and the mRNA or cDNA for cDNA library construction come from any suitable tissue, cell and library containing corresponding mRNA or cDNA. For example, the NGF encoding sequence can be obtained from the NGF cDNA library by PCR; The BDNF encoding sequence can be obtained from a cDNA template by RT-PCR in which the cDNA template is obtained from RNA extracted from cerebral tissue by reverse transcription; The nucleotide sequence encoding PTD can be obtained by artificial synthesis in which host preference codons may be used so as to increase the expression amount of the encoding sequence. An example encoding nucleotide sequence for PTD in Antp and corresponding amino acid sequence thereof is shown as below, in which preference codons for *Escherichia coli* (*E. coli*) is utilized:

5'-TCCGGCAGACAAATCTGGTTCCAATTCCAAAACAGGAGGATGAAGTGGAAAAAATGC-3'
S G R Q I K I W F Q N R R M K W K K C

Mutation, deletion, insertion and connection with other nucleotide sequence can be made to the polynucleotide by common methods in the field if desired. The fusion of the nucleotide sequences encoding NTs and PTD can be carried out by common methods in the field provided that no change will occur to each open-reading frame. For example, restriction enzyme recognition sites may be introduced into a pair of primers used for PCR amplification so that the sequences can be digested by restriction enzyme, leaving adhesive end for connecting with sequence having complementary adhesive end to form a gene encoding the fusion protein. The nucleotide encoding the fusion protein is cloned into various expression vectors using common methods in the filed when the correctness of the nucleotide sequence of the fusion protein has been verified by sequencing. For standard procedure of molecular clone, please refer to the description of J.Sambrook *et al* (J. Sambrook, et al. Molecular Cloning: A Laboratry Manual. Cold Spring Harbor Lab(CSHL), 2001) .

The expression of the NTs-PTD fusion protein provided in the present invention can be carried out in various expression systems. The encoding sequence for fusion protein, according to one embodiment of the present invention, can be cloned to a prokaryote expression system to form a pNTs-PTD which may be expressed in prokaryotic cell such as *E.coil*. Alternatively, the expression vectors can be a yeast expression vector system, an animal cell expression vector or a plant cell expression vector. The expression vectors and corresponding host cells are commercially available. For example, the *E.coli* pET expression system serial can be obtained from Novagen company, the yeast pPIC expression system serial and the pcDNA expression system serial for mammalian cell can be obtained from Invitrogen company. Host cell may be chosen based on the desired advantages. For example, *E.coil* facilitates the operation procedures and provides high amount of a expressed protein; mammalian cell provides correct post-translation glucosylation, but with relatively low level of expression; yeast cell incorporates both advantages associated with *E. coli* and mammalian cell. In the present invention, renatured fusion protein may form intermolecular disulfide bond without the need for post-translation glucosylation, so the *E. coli* is for a preferable host cell used for the present invention.

Any suitable method in the art can be used to transform an expression system containing nucleonic acid sequence encoding NTs-PTD to a host cell, including, but are not limited to, chemical transformation and electroporation. Cell lysis followed by PCR and plasmid DNA sequencing can be used to determine whether the host cell is successfully transformed.

A large number of NTs-PTD fusion proteins can be obtained through culturing the host cells, such as recombinant bacteria, recombinant yeast, or recombinant eukaryotic cell and so on which contains the nucleotide sequence described above in the invention. For example, the fusion proteins can be largely obtained by shaking culture the *E. coil* BL21 containing the recombinant prokaryotic vector under optimized conditions. The fusion proteins may be then purified using liquid chromatography and other chromatographic techniques.

In one example, the purified BDNF-PTD/Antp fusion protein can significantly promote the growth of the PC12/trkB neuron *in vitro,* which demonstrates that the fusion protein maintains the neurotrophic activity of BDNF. In another example, the BDNF-PTD/Antp fusion protein in brain can be detected four hours after the fusion protein is administrated through caudal vein of mice by methods known to the person skilled in the art.

NTs are promising means for treating many clinical diseases especially neurodegenerative diseases in central nervous system (CNS). However, NTs can not cross BBB into brain and function therein when administrated through peripheral vascular or muscle injection. In the present invention, the NTs-Antp fusion protein can deliver NTs to BBB after being administrated through vessels due to the protein transduction domains carried by NTs, so the fusion protein NTs-Antp in the present invention can play a neurotrophic function after entering cerebral parenchyma via peripheral vein delivery.

NTs containing the protein transduction domains may function in cerebral parenchyma via peripheral vein administration, so that the "spectrum" of diseases that can be treated by NTs will be significantly broadened, which including, but are not limited to, nerve injury caused by cerebral trauma or stroke, Parkinson's disease, nervous system congenital disease caused by nervous system hypoplasia, encephalatrophy, postencephalitis, anoxic encephalopathy, neurasthenia and neoplastic headache, epilepsy, vascular dementia, early Senile Dementia, vertebral injure, nerve replantation, polyneuritis, diabetes mellitus peripheral nervous lesion, never rupture degeneration, sciatic nerve injure, facioplegia, optic nerve lesion and on the like.

For illustrative purpose, the present invention will be described in detail in the following examples. However, it is not intended to limit the invention to the preferable examples provided below. Many changes or modifications can be made to the present invention without departing from the scope of the invention which is defined by the appended claims. The present invention is intended to cover all the changes and modifications and equivalents thereof.

### Example 1: Structural design of the BDNF-PTD/Antp fusion protein

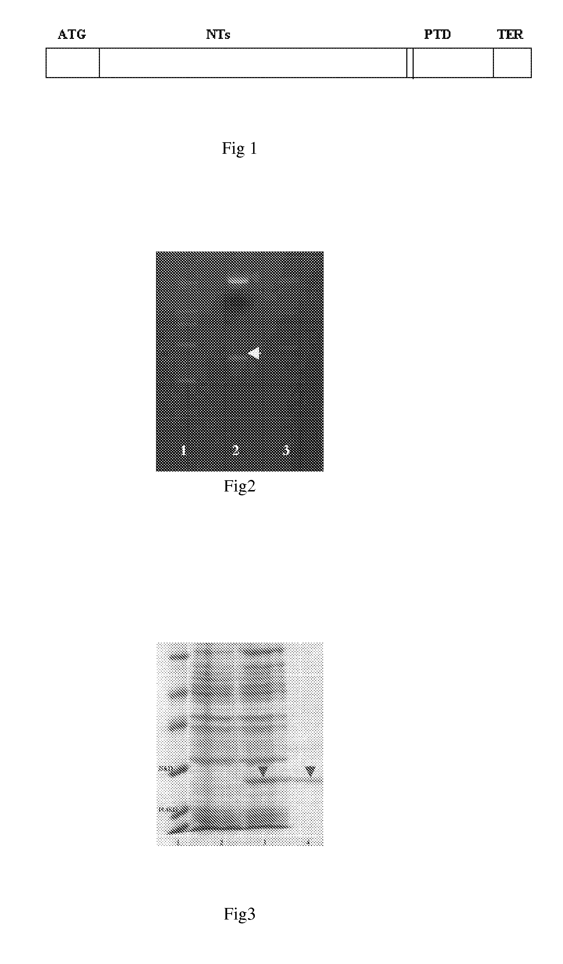

Referring now to figure 1, in which the structure of the encoding DNA sequence for BNDF-PTD/Antp fusion protein is schematically shown. In figure 1, ATG encodes Methionine; sequence encoding protein transduction domain is shown as PTD; NTs means the nucleotide sequence encoding the mature protein of family members of human neurotrophins; TER is the terminator codon. Methionine was added before the maturation region of neurotrophins. PTD/Antp is located at the C-terminal of BDNF and connects to BDNF through intervening glutamic acid. DNA sequence encoding BDNF-PTD/Antp fusion protein thus obtained has a structure shown in Figure 1.

### Example 2: Amplification of the gene for BDNF-PTD/Antp fusion protein and construction of the prokaryotic expression vector

According to the nucleotide sequence encoding BDNF mature protein reported by Genebank and the structure of the BDNF-PTD/Antp fusion protein identified above, the primers for amplification of the gene of the BDNF-PTD/Antp fusion protein are designed as follows:
Primer I with sequence indicated in SEQ ID NO: 3 is as follows:
   5'-GCCATATGCACTCTGACCCTGCCCGCCGA-3'(with NdeI site underlined)
Primer II with sequence indicated in SEQ ID NO: 4 is as follows: 
5'-GCCTCGAGCTAGCATTTTTCCACTTCATCCTCCTGTTTGGAATTGGA
ACCAGATTTGTCTGCCGGATCCCCTTTTAATGGTCAATGTACATAC-3'
(with XhoI site underlined)

DNA encoding for BDNF-PTD/Antp fusion protein is amplified by PCR with plasmid popeMPBDNF( from American ATCC) containing full-length cDNA of human BDNF as a template. PCR was performed under the following conditions: 3 min of predenaturation at 94°C, 45 seconds of denaturation at 94 °C, annealing for 1 min at 58°C, extension for 1min at 72°C, 35 cycles, finished by extension for 10min at 72°C.The amplification product is analyzed by 1.5% agarose gel electrophoresis. And then the target gene fragment is extracted using gel extraction kit. After digested by the restriction enzymes, the PCR products were subcloned into pET30(a) ( available from Novagen,USA) by the way of the NdeI-XhoI sites located at the terminals of the PCR product. The ligation and the transformation are carried out according to the procedures describe in *molecular clone.* Referring now to Figure 2, the vector containing target gene is analyzed with restriction enzymes, resulting in the vector and target gene fragment. Lane 1 and 3 are Marker with low and high molecular weight respectively. In Lane 2, the upper arrow designates the expression vector plasmid fragment, the lower arrow designates the nucleic acid fragment encoding BDNF-PTD/Antp. The target gene sequence is verified by sequencing. Results show the nucleic acid fragment has a sequence listed as SEQ ID NO: 2.

### Example 3: Expression of the BDNF-PTD/Antp fusion protein

*E.coli* BL21(DE3)plys serves as a competent cell, which is transformed with recombinant plasmids encoding the BDNF-PTD/Antp fusion protein. Transformed *E.coli* are grown on the culture dish coated with kanamycin (50mg/mL) and chloromycetin (34mg/mL). Several single positive clones are randomly chosen and then grown in 200 mL LB medium containing kanamycin ( 50mg/mL ) and chloromycetin(34mg/mL) at 37°C overnight. A portion of the culture is transferred to a 1000 mL LB medium with the same concentrations of Kanamycin and Chloromycetin as above and incubated until OD₆₀₀ is equal to 0.6. The protein expression was induced by adding IPTG to a final concentration of 0.1mmol/L, then incubated for another 5 hours. Then cells were collected by centrifugation at 5000prm/min for 15min at 4°C.

### Example 4: Purification of the BDNF-PTD/Antp fusion protein and Western blot analysis

Induced cells were harvested and then lysed in a cell lysis buffer(8M urea, 0.01M Tris-Cl, PH8.0, 0.1M NaH₂PO₄) in a ratio of 10 g/ml and sonicated. The fusion proteins are thus dissolved or distributed in the solution, which can be separated from the cells by centrifugation at 10000×g for 30 min at 4°C as a supernatant. The supernatant is past through a Q-Sepharose ion exchange column, followed by purified with a Sephacryl gel filter column. A fusion protein with a purity of more than 99.5% is obtained. The native fusion proteins were obtained after dialyzing and renaturation in PBS (0.1 M) containing 0.1 M arginine.

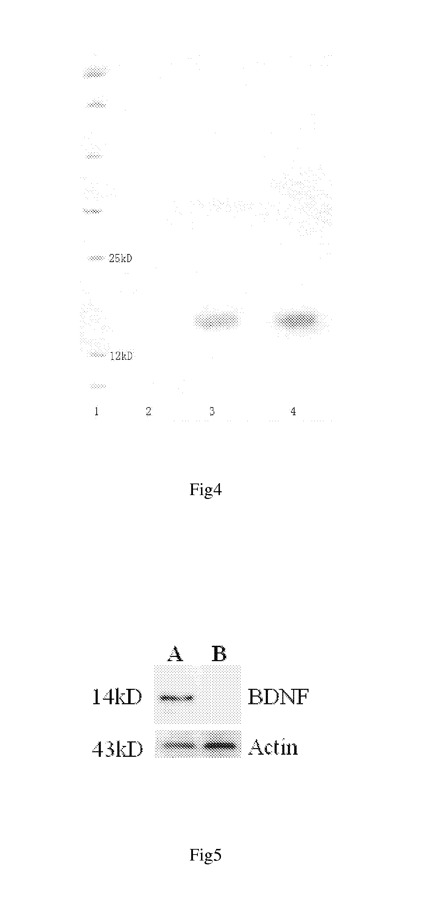

Figure 3 shows the result of the SDS-PAGE analysis followed by Coomassie Brilliant blue staining. In Figure 3, Lane 1 is loaded with protein molecular weight marker, lane 2 is loaded with supernatant obtained from the cell lysis solution without induction, lane 3 is loaded with supernatant obtained from the cell lysis solution after induction, lane 4 is loaded with purified fusion protein. The band corresponding to 18 kDa in lane 4 shows that the fusion protein is expressed in the cell after induced by IPTG. While at the corresponding position in the control lane, no band is observed. The purified proteins are then transferred to nylon membranes and probed with a rabbit anti-human BDNF antibody. The result of Western blot was shown in the figure 4 in which lane 1 is loaded with protein molecular weight marker, lane 2 is loaded with supernatant of the cell lysis solution without induction, lane 3 is loaded with supernatant of the cell lysis solution after induction and lane 4 is loaded with the purified fusion protein. A specific band is appeared on the site at 18kD which is the same as that in the SDS-PAGE analysis. The result in western blot indicates that BDNF-Antp fusion protein was successfully expressed and purified. In another experiment, 4 µg of recombinant BDNF-Antp fusion protein and recombinant BDNF are respectively administrated through caudal vein of mice, then a Western blot analysis of cerebral tissue is proceeded 4 hours after the administration. The BDNF protein can be obviously detected in the animal tissue treated with the BDNF-Antp fusion protein. The results are shown in Figure 5, in which Group A represents mice treated with BDNF-Antp, Group B represent mice treated with BDNF. Comparing to the control group, group A which is administrated with BDNF-Antp contains higher level of BDNF.

### Example 5: Assay of bioactivity for BDNF-PTD/Antp fusion protein to promote the proliferation of PC 12/trkB chromaffinoma cell line

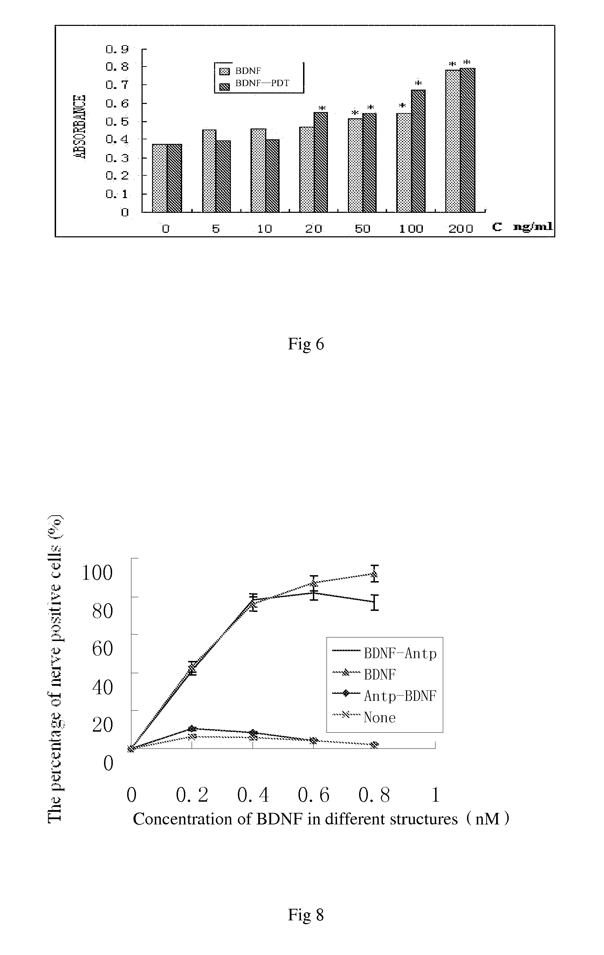

MTT is used to assess the biological activity of BDNF-PTD/Antp fusion protein. Different concentrations of renaturated genetically recombinant protein BDNF-PTD/Antp and a positive control protein BDNF are added into the culture solution for PC12/trkB chromaffinoma cell line, in both cases cell proliferations are promoted. Statistic analysis shows the difference is significant when comparing to negative control. Further, the biological activity of BDNF-PTD/Antp was better than BDNF. The result is shown in figure 6, which indicates that, PTD fused at the C-terminal of BDNF has no effect on the formation of both dimer and structure of N-terminal of BDNF, moreover, the BDNF biological activity was maintained and even enhanced.

### Example 6: BDNF-PTD/Antp fusion protein capable of crossing BBB in vivo

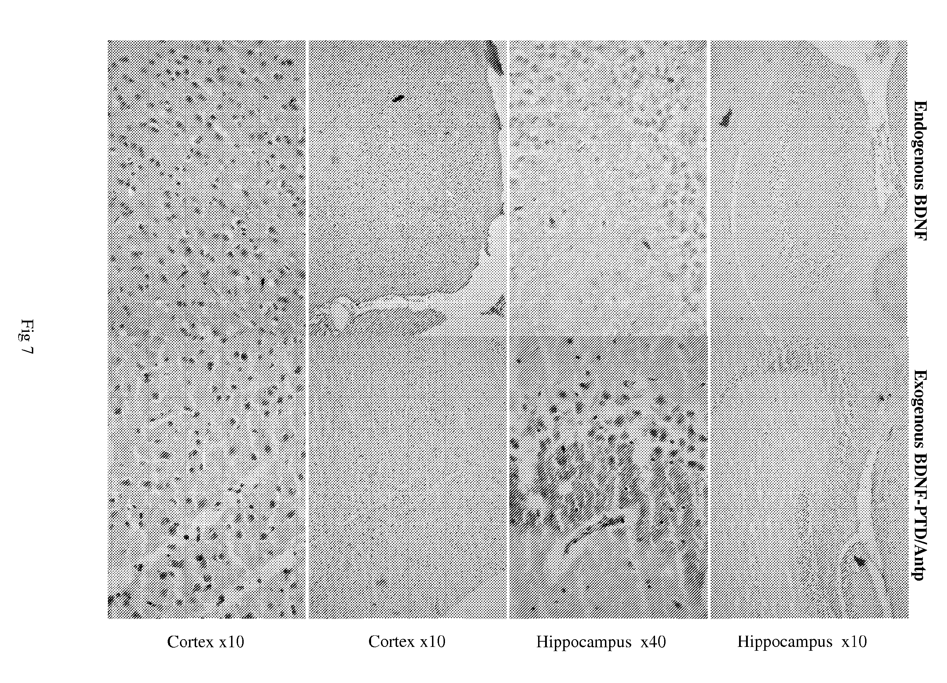

4 µg of BDNF-PTD/Antp recombinant protein and the control recombinant protein BDNF are respectively administrated to caudal vein of 20-25g mice. 4 hours later, the mouse brains are isolated, and slices of brain are prepared for the analysis of the distribution of delivered BDNF-PTD with an immunohistochemical method. In BDNF-delivered group, the detected tissue distribution of BDNF is consistent with that of the endogenic BDNF as reported, which shows that extrinsic BDNF administered through peripheral vascular route can not penetrate BBB to the brain. However, in BDNF-PTD/Antp-delivered group, strong positive expression is widely distributed in the brain, especially at the hippocampus and the striaturn cortex, as shown in Figure 7, which indicates that PTD can play a function of transduction so as to enable BDNF to cross BBB. The indications of NTs will be greatly extended due to a PTDs fusion enable NTs across BBB into the brain through a peripheral vascular route, which makes NTs treat CNS neurodegenerative diseases.

### Example 7: Comparison of the bioactivity PTD-BDNF and BDNF-PTD

Antp-BDNF is prepared using the same method as that of examples 2-4 described above. The PTD/Antp is linked to the N- or C-terminal of BDNF to make PTD-BDNF and BDNF-PTD as described above. MTT is used to assess the biological activity of BDNF-Antp and Antp-BDNF fusion protein. Different concentrations of the renaturated recombinant fusion protein BDNF-Antp, Antp-BDNF and the positive control recombinant protein BDNF were added into the culture media for PC12/trkB chromaffinoma cell line. Promotion of cell proliferation is observed in the BDNF-Antp group and BDNF group. The difference comparing to the negative control is significant. While the Antp-BDNF group activity of promotion cell proliferation had no significant difference comparing with the negative control group. Figure 8, in which the X-axis represents the concentration (nM) of different structure form of BDNF, and the Y-axis represents the percent of the neuron-like positive cells, shows that BDNF with naked N-terminal can more effectively promote the PC12-trkB cell proliferation, and the N-terminal of BDNF is perhaps the most important functional domain for cell proliferation.

### Example 8: Neuroprotection in cerebral brain ischemia following intraperitoneal injection of the BDNF-Antp fusion protein

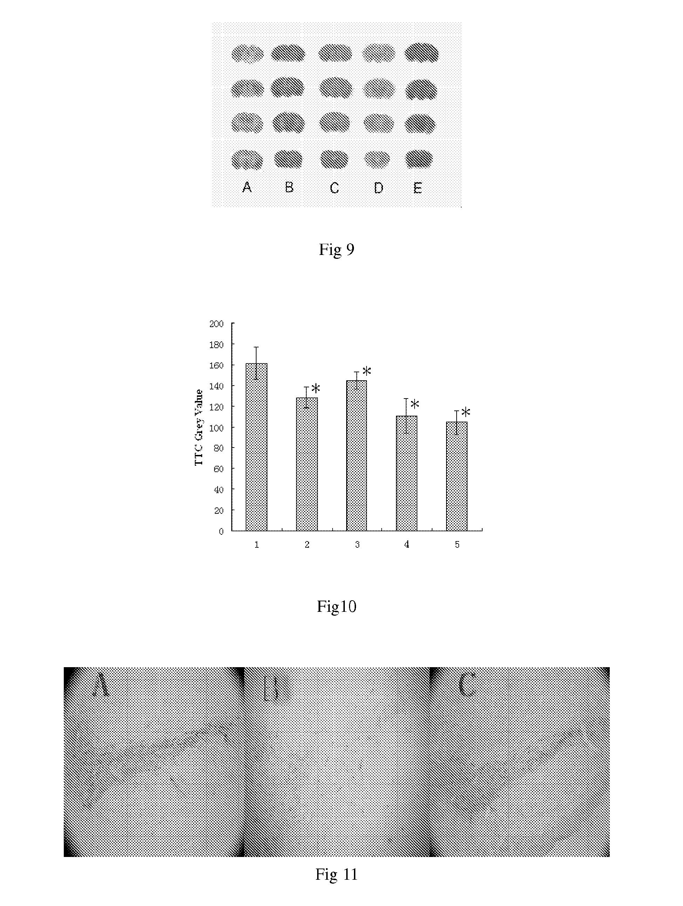

A cerebral ischemia model in Gerbillinae is prepared. BDNF-Antp is intraperitoneally administrated to the gerbillinaes at different time. TTC staining for the brain slices is carried out after gerbillinaes are killed (staining results shown in Fig.9), and followed by gray scanning (results shown in Figure 10). Figure 9 shows that BDNF-Antp can protect neuron against necrosis caused by frontal cerebral ischemia, wherein A represents the group in which physiological saline is intraperitoneally injected when ischemia occurs; B represents the group in which BDNF-Antp is intraperitoneally injected 2 hours before the ischemia occurs; C represents the group in which BDNF-Antp is intraperitoneally injected 0 hour before the ischemia occurs; D represents the group in which BDNF-Antp is intraperitoneally injected 1 hour after the ischemia occurs; and E represents the group of normal brain. Figure 10 shows the gray comparison after TTC staining. There is significant difference between the group of administration with BDNF-Antp at different time and the group of administration with saline, which indicates that BDNF-Antp could protect the neuron from necrosis after crossing BBB. In Figure 10, group 1 represents the group in which physiological saline is injected when ischemia occurs; group 2 represents the group in which BDNF-Antp is injected when ischemia occurs; group 3 represents the group in which BDNF-Antp is injected 1 hour before the ischemia occurs; D represents the group in which BDNF-Antp is injected 2 hours before the ischemia occurs; and E represents the group of normal brain.

The statistics analysis reveals that BDNF-Antp is able to protect neuron from necrosis caused by cerebral ischemia and the protection level is closely related with the administration time of BDNF-Antp. The results show the neuroprotection in cerebral brain ischemia following intraperitoneal injection of the BDNF-Antp fusion protein.

### Example 9: Intraperitoneal injection of BDNF-Antp fusion protein prevents MPTP-induced dopamine depletion in a murine model of Parkinson's disease.

Parkinson's disease model in mice induced by MPTP (1-methyl-4-phenyl-1,2,3,6-tetrahydropyridine, MPTP) is prepared. BDNF-Antp treatment group obviously lessened the damage to the dopamine neuron comparing to that in the control group. Figure 11 shows the immunohistochemistry of tyrosine hydroxylase (a positive label of dopamine neuron) in mesencephalic substantia nigra of C57BL mouse. In Figure 11, group A represents the control group in which a normal amount of dopamine neuron exists in the mesencephalic substantia nigra; Group B is a Parkinson's disease model group induced by intraperitoneal injection of MPTP, in which dopamine neuron in mesencephalic substantia nigra is obviously decreased. Group C is a treatment group, in which BDNF-Antp fusion protein (5µg/25g mice) was i.p. delivered 2 hours before administration of MPTP, and the dopamine neuron in the mesencephalic substantia nigra is dramatically increased compared to that in group B. The results indicate that BDNF-Antp fusion protein prevents MPTP-induced dopamine depletion in a murine model of Parkinson's disease, which allows BDNF-Antp be used to treat Parkinson's disease.

### Example 10: Protein transduction function of Antp-PTD mutant with 70% homology with Antp-PTD)

Nucleotide sequences encoding amino acid sequence of Antp/PTD undergo randomly mutation. PCR as describe in example 2 is used to clone the gene encoding BDNF-Antp/PTD mutant, in which the amino acid sequence of Antp/PTD mutant is about 70% homology with the native Antp/PTD. The fusion protein of BDNF-Antp/PTD mutant is prepared by the method mentioned in examples 3 and 4. The effect for BDNF-Antp/PTD mutant crossing BBB is demonstrated by the method of example 6. The results show that there is a strong positive expression in many position of the animal's brain especially in the hippocampus and striaturn when the BDNF-Antp/PTD mutant is administrated through caudal vein, which indicates thatthe Antp/PTD mutant has the same protein transduction function as that of native one. In another example, an Antp/PTD mutant having an amino acid homology of 85% with native one is demonstrated to have protein transduction function of PTD too.

### Example 11: Promotion of cell proliferation by BDNF mutant (70% of amino acid homology with mature BDNF)

Nucleotide sequences encoding BDNF undergo randomly mutation. PCR as described in example 2 is used to clone the gene encoding the BDNF mutant. The mutant is prepared by the method mentioned in examples 3 and 4. The biological activity of BDNF mutant protein (70% of amino acid homology with mature BDNF) was analyzed by the method in the example 5. Different amounts of the recombination BDNF mutant protein (70% of amino acid homology with mature BDNF) and positive control (recombination BDNF) are added into the culture solution of PC12/trkB chromaffinaoma cell line. Promotions of cell proliferation in both groups are observed. Compared with the negative group (without mature BDNF or mutant BDNF), the proliferative difference is significant by statistics analysis, which indicates that the mutant protein possesses the same biological activity as native mature BDNF. In another example, the BDNF mutant having an amino acid homology of 85% with mature BDNF is demonstrated to have the same biological activity as that of non-mutant BDNF.

### Example 12: Biological activity of BDNF (mutant)-Antp/PTD (mutant) fusion protein

BDNF (mutant)-Antp/PTD (mutant) fusion protein is prepared using the methods of example 10 and 11. In the fusion protein, the BDNF mutant has an amino acid homology of 70% with native BDNF, and the Antp/PTD mutant has an amino acid homology of 70% with native Antp/PTD. The in vivo experiments show that the BDNF(mutant)-Antp/PTD(mutant) fusion protein is able to protect the neuron apoptosis resulted from ischemia and the protection extent is closely related to the administration time before or after ischemia, which indicates that BDNF (mutant)-Antp/PTD mutant fusion protein may be used to restore neuron functions after stroke.

In another example, the BDNF (mutant)-Antp/PTD (mutant) fusion protein in which the BDNF mutant has an amino acid homology of 85% with native BDNF and Antp/PTD mutant has an amino acid homology of 85% with native Antp/PTD is demonstrated to have a possibility to restore neuron functions after stroke.

## Claims

1. A fusion protein for carrying neurotrophins across blood-brain barrier, comprising:
a first region having amino acid sequences which has at least about 75% homology with that of mature neurotrophin; and
a second region having amino acid sequences which has at least about 70% homology with that of native protein transduction domain
wherein said second region is located at the carboxyl terminal of said first region.

2. The fusion protein for carrying neurotrophins across blood-brain barrier according to claim 1, wherein said first region has amino acid sequences having at least about 85% homology with that of mature neurotrophin, and said second region has amino acid sequences having at least about 85% homology of that of native protein transduction domain.

3. The fusion protein for carrying neurotrophins across blood-brain barrier according to claim 1, wherein said first region is human nerve growth factor, brain-derived neurotrophic factor, neurotrophin 3, or neurotrophin 4/5.

4. The fusion protein for carrying neurotrophins across blood-brain barrier according to claim 1, wherein said second region is from the PTD of a homologous heterotypic protein Antp originated from fruit fly, from the PTD of pVEC of type I membrane protein Cadherin from mice vascular endothelial cells, or from the PTD of HIV transactivator of transcription Tat.

5. The fusion protein for carrying neurotrophins across blood-brain barrier according to claim 1, wherein said second region is immediately adjacent to said first region, or connects to said first region through intervening amino acids.

6. The fusion protein for carrying neurotrophins across blood-brain barrier according to claim 1, wherein said first region is human brain-derived neurotrophic factor, said second region is homologous heterotypic protein Antp originated from fruit fly, and the fusion protein has a amino acid sequence presented in SEQ ID NO:1.

7. A nucleotide sequence encoding the fusion protein of claim 6, wherein said nucleotide sequence has a sequence presented in SEQ ID NO:2 or a complementary sequence thereof.

8. An expression vector comprising the nucleotide sequence of claim 7.

9. A host cell comprising the expression vector of claim 8.

10. Usage of fusion protein of claim 1 in the preparation of medicines for treating neurotrophins related diseases.


Sequence Listing

<110> Southern Medical University Zhujiang Hospital

<120> A fusion protein for carrying neurotrophin across the blood-brain barrier,
encoding gene and uses thereof

<130> P41350

<140> 07 800 858.8
<141> 2007-09-04

<150> CN200610122393.9
<151> 2006-09-25

<160> 4

<210> 1
<211> 138
<212> PRT
<213> E. coli
<400> 1
Met His Ser Asp Pro Arg Arg Arg Gly Glu Leu Ser Val Cys Asp Ser
1               5                   10                  15
Ile Ser Glu Trp Val Thr Ala Ala Asp Lys Lys Thr Ala Val Asp Met
1               5                   10                  15
Ser Gly Gly Thr Val Thr Val Leu Glu Lys Val Pro Val Ser Lys Gly
1               5                   10                  15
Gln Leu Lys Gln Tyr Phe Tyr Glu Thr Lys Cys Asn Pro Met Gly Tyr
1               5                   10                  15
Thr Lys Glu Gly Cys Arg Gly Ile Asp Lys Arg His Trp Asn Ser Gln
1               5                   10                  15
Cys Arg Thr Thr Gln Ser Tyr Val Arg Ala Leu Thr Met Asp Ser Lys
1               5                   10                  15
Lys Arg Ile Gly Trp Arg Phe Ile Arg Ile Asp Thr Ser Cys Val Cys
1               5                   10                  15
Thr Leu Thr Ile Lys Arg Gly Ser Gly Arg Gln Ile Lys Ile Trp Phe
1               5                   10                  15
Gln Asn Arg Arg Met Lys Trp Lys Lys Cys
1               5                   10

<210> 2
<211> 417
<212> DNA
<213> E. coli
<400> 2
atgcactctg accctgcccg ccgaggggag ctgagcgtgt gtgacagtat tagtgagtgg 60
gtaacggcgg cagacaaaaa gactgcagtg gacatgtcgg gcgggacggt cacagtcctt 120
gaaaaggtcc ctgtatcaaa aggccaactg aagcaatact tctacgagac caagtgcaat 180
cccatgggtt acacaaaaga aggctgcagg ggcatagaca aaaggcattg gaactcccag 240
tgccgaacta cccagtcgta cgtgcgggcc cttaccatgg atagcaaaaa gagaattggc 300
tggcgattca taaggataga cacttcttgt gtatgtacat tgaccattaa aaggggatcc 360
ggcagacaaa tctggttcca attccaaaac aggaggatga agtggaaaaa atgctag 417

<210> 3
<211> 19
<212> DNA
<213> Artificial Sequence
<220>
<223>sequence designed based on fusion protein genes, and used as a primer for
amplification of DNA sequence of fusion protein
<400> 3
gccatatgca ctctgaccct gcccgccga

<210> 4

<211> 95
<212> DNA
<213> Artificial Sequence
<220>
<223> sequence designed based on fusion protein genes, and used as a primer for
amplification of DNA sequence of fusion protein
<400> 4
gcctcgagct agcatttttt ccacttcatc ctcctgtttt ggaattggaa ccagatttgt  60
ctgccggatc cccttttaat ggtcaatgta catac 95